# EUROPEAN PATENT APPLICATION

(11) **EP 3 760 169 A1**
(43) Date of publication of application: **06.01.2021**
(21) Application number: 20183554.3
(22) Date of filing: 01.07.2020
(51) Int. Cl.: A61F 5/01, A61F 5/058

(54) **THUMB BRACE**

(30) Priority: 05.07.2019 NL 2023449
(71) Applicant: WE Design Beheer B.V., 6814 EN Arnhem (NL)
(72) Inventor: Engelshoven, Wouter Robin, Arnhem (NL)
(74) Representative: Nederlandsch Octrooibureau

(57) **Abstract**

A thumb brace (1) comprising a wire frame (2) having a palmar wire section (3) configured to extend in lateral direction from a web point (W) thereof between a thumb (T) and index finger (I) along a palmar region (4) toward an ulnar side of the hand. The palmar wire section (3) is connected to an ulnar wire section (7) and radial wire section (8), the ulnar wire section (7) being configured to extend from the ulnar side along a dorsal ulnar region (9) of the hand and wherein the radial wire section (8) is configured to extend from the web point (W) along a first dorsal interosseous region (10) of the hand. The wire frame (2) further comprises a ring shaped thumb wire section (11) for receiving the thumb (T), and wherein the palmar wire section (3) comprises a palm engaging portion (5) that widens in proximal-distal direction.

## Description

### Field of the invention

The present invention relates to a thumb brace/orthosis, in particular a frame/wire-based thumb brace for correcting/supporting cases of CMC joint arthritis or MP hyperextension for example.

### Background art

There are known thumb braces for correcting or supporting various thumb problems, wherein the thumb braces are made of a wire/frame which is shaped to snugly fit around a patient's thumb for support thereof. Such wire-based thumb braces are advantageous in that they are waterproof and provide a low profile and sleek design that allows the thumb brace to not interfere with day to day activities.

However, the known wire based thumb braces primarily focus on resolving issues of the thumb only but in doing so may unnecessarily impede or discourage particular movements of the fingers and/or postures of the hand and finger that a patient is still able to tolerate. For example, known wire-based thumb braces may discourage or impede natural palm posture associated when pinches fingers.

### Summary of the invention

The present invention aims to provide an improved thumb brace, in particular a wire/frame based thumb brace, to support the thumb for cases of e.g. MP hyperextension, CMC joint arthritis and/or excessive CMC flexion whilst still allowing for natural palm and finger positions/postures that a patient is still able to tolerate.

According to the present invention, a thumb brace of the type defined in the preamble is provided comprising a wire frame having a palmar wire section which is configured to extend in lateral direction from a web point thereof between a thumb and index finger of a hand along a palmar region toward an ulnar side of the hand, wherein the palmar wire section is connected to an ulnar wire section and a radial wire section, the ulnar wire section being configured to extend from the ulnar side in lateral direction along a dorsal ulnar region of the hand and wherein the radial wire section is configured to extend from the web point along a first dorsal interosseous region of the hand. The wire frame further comprises a ring shaped thumb wire section configured to receive the thumb, and wherein the palmar wire section comprises a palm engaging portion that (locally) widens in proximal-distal direction.

In light of the present invention, the (local) widening of the palm engaging portion provides improved distributed support to the palm such that pinching the thumb and index finger is facilitated, allowing patients to produce a natural round/oval shape between the thumb and index fingers whilst pinching. The thumb wire section provides the required orthopaedic support for cases of e.g. MP hyperextension, CMC hyperflexion or CMC joint arthritis.

In an advantageous embodiment, the palm engaging portion is a concave palm engaging portion, so that in addition to the aforementioned distributed support, a natural concave posture/shape of the palm of the hand is possible to further improve a patient's ability to pinch the thumb and index finger naturally and allow for a round/oval shape between the thumb and index finger whilst pinching.

According to an exemplary embodiment, the palm engaging portion may comprises two wire parts that are connected parallel/side-by-side to one another at an ulnar side end and at an opposing radial side end of the palmar wire section, wherein the two wire parts separate or bend outwards in proximal-distal direction between the ulnar and radial side ends. Through this embodiment is it possible to provide distributed support to the palm of the hand by solely using a wire frame, i.e. there is no need for a separate pad-like component to provide support to the palm. As a result, a sleek design is maintained, keeping the palm as free as possible, provide sufficient air circulation and maintain hygiene.

### Short description of drawings

The present invention will be discussed in more detail below, with reference to the attached drawings, in which
Figure 1 shows a volar view of a prior art thumb brace;
Figure 2 shows a lateral-radial view of a thumb brace according to an embodiment of the present invention;
Figure 3 shows a volar view of a thumb brace according to an embodiment of the present invention;
Figure 4 shows a lateral-radial view of a thumb brace according to another embodiment of the present invention; and wherein
Figure 5 shows a lateral-ulnar view of a thumb brace according to an embodiment of the present invention.

### Description of embodiments

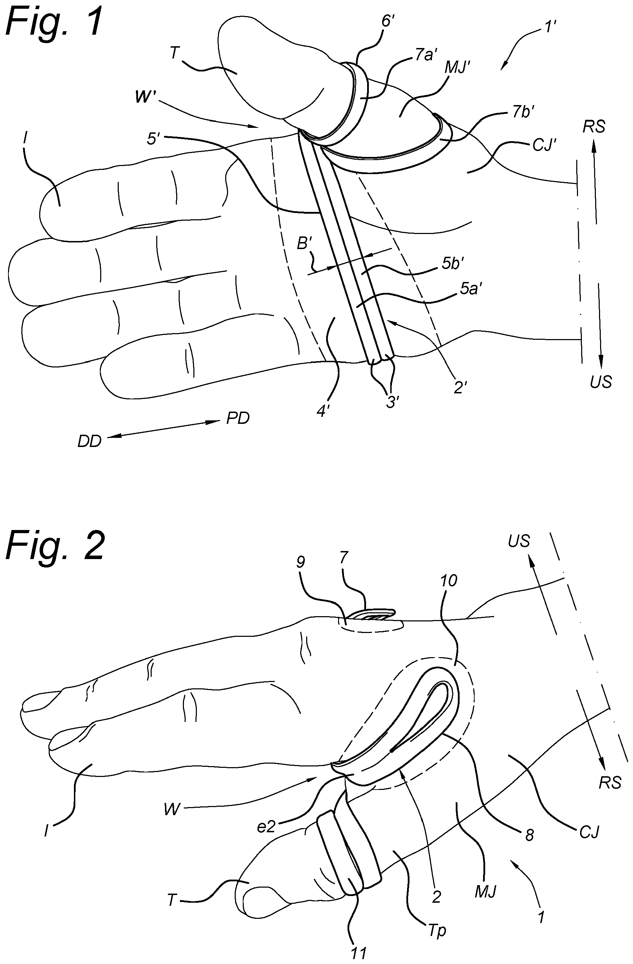

Figure 1 depict a thumb brace 1' as known in the prior art. The thumb brace 1' is made from a substantially rigid wire frame 2' that is shaped/bent to snugly engage a hand of a patient and provide support to the thumb T. As depicted, a substantially straight palmar wire section 3' is provided that is configured to extend in lateral direction from a web point W' thereof, between the thumb T and the index finger I, along a palmar region 4' of the hand toward an ulnar side US of the hand. Note that the "ulnar side" US corresponds to the position of the ulnar of a lower arm. The aforementioned web point W' may as such be associated with the position of the radius of the lower arm, i.e. the web point W' may correspond to a "radial side" RS of the lower arm. Therefore, the palmar wire section 3' extends from a radial side RS to an ulnar side RS of the hand along the palmar region 4' thereof.

Affixed to the palmar wire section 3' is a thumb wire section 6' which is configured to receive a thumb T to immobilize motion thereof, such as immobilizing the CMC and/or MCP joint of the thumb T according to orthopaedic needs. The thumb wire section 6' comprises a proximal phalanx portion 7a', corresponding to the proximal phalanx bone, as well as a first metacarpal portion 7b', corresponding to the first metacarpal bone, thereby immobilizing the MCP joint MJ' and CMC joint CJ' .

Although not shown, the palmar wire section 3' is configured/shaped to extend along the ulnar side US of the hand toward a dorsal side (not shown) of the hand. The palmar wire section 3' is further configured to extend along the web point W', at a radial side RS of the hand toward the dorsal side of the hand. This allows the thumb brace 1' to be secured/affixed to the hand and to provide orthopaedic stability to the thumb T as required.

In Figure 1 it is further depicted that the palmar wire section 3' comprises a palm engaging portion 5' have a constant width B' in a proximal-distal direction PD-DD of the hand, wherein the proximal-distal direction may be viewed as a longitudinal/lengthwise direction of the hand. The consent width B' is determined by two wire parts 5a', 5b' that are lengthwise connected in parallel, i.e. side-by-side, to one another. However, as the two wire parts 5a', 5b' have a relative narrow width, the width B' is also relatively small so that the palm engaging portion 5' tends to "cut" into the palm of the hand when, e.g., a patient wishes to pinch the thumb T and index finger I together.

In light of the above, there is a need for a thumb brace that allows patients to easily pinch the thumb T and index finger I whilst maintaining good overall fixture of the thumb brace to the hand as well as maintaining orthopaedic support and comfort to the thumb T.

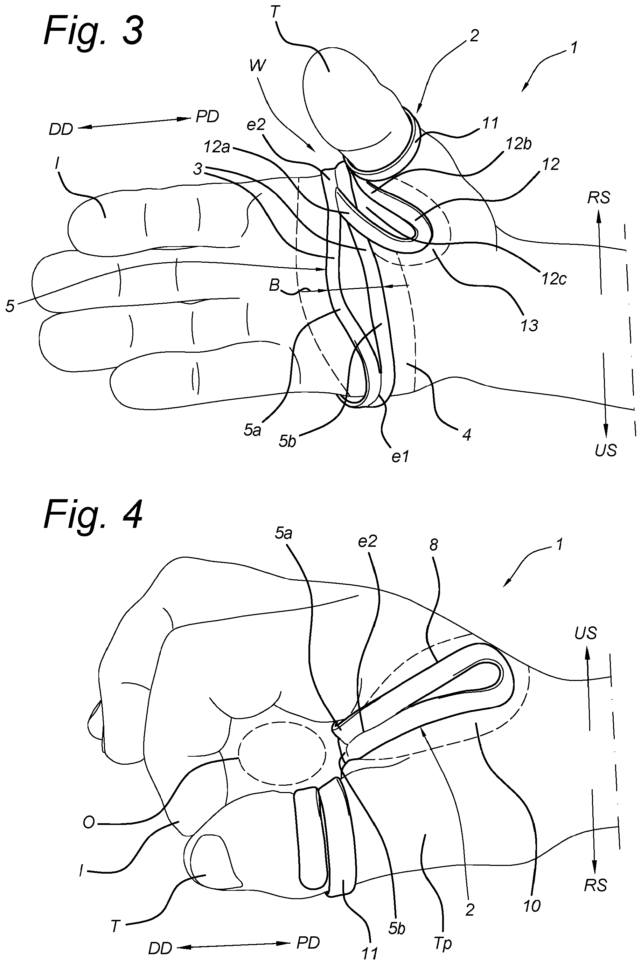

A thumb brace that meets at least in part the above need is depicted in Figure 2 and 3, showing a lateral-radial view and a volar view, respectively, of a thumb brace 1 according to an embodiment of the present invention. In the embodiment shown, the thumb brace is 1 is made of a wire frame 2 and comprises a palmar wire section 3 which is configured/bent to extend in lateral direction from a web point W thereof between a thumb T and index finger I along a palmar region 4 of the hand toward an ulnar side US of the hand. The palmar wire section 3 is connected to an ulnar wire section 7 and a radial wire section 8, which are located at the ulnar side US and radial RS of the hand respectively.

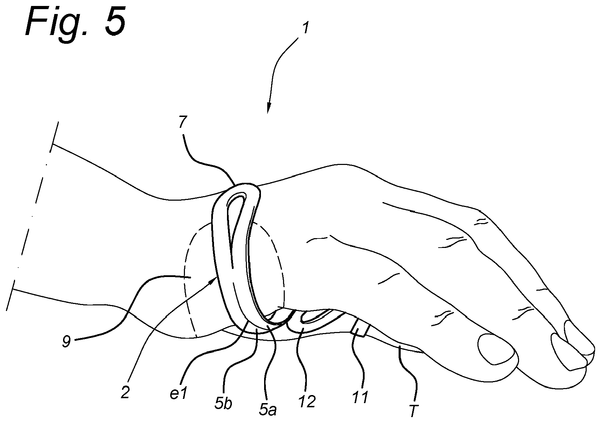

The ulnar wire section 7 is configured/bent to extend from the ulnar side US of the hand in lateral direction along a dorsal ulnar region 9 of the hand (also see Figure 5) and wherein the radial wire section 8 is configured/bent to extend from the web point W along a first dorsal interosseous region 10 of the hand.

The wire frame 2 further comprises a ring shaped thumb wire section 11 which is configured/bent to receive the thumb T to provide support for cases of, e.g., MP hyperextension, CMC joint arthritis and/or excessive CMC flexion. Advantageously, the thumb wire section 11 is configured to receive a proximal phalanx portion Tp of the thumb T to provide good support of the MCP joint and/or CMC joint according to orthopaedic needs.

As further shown in Figure 3, the palmar wire section 3 comprises a palm engaging portion 5 that widens in the indicated proximal-distal direction PD-DD, i.e. in longitudinal/lengthwise direction of the hand. That is, the widening of the palm engaging portion 5 is a local widening along the palmar region 4, wherein a varying width B of the palm engaging portion 5 increases to a maximum and subsequently decreases as seen from the radial side RS to the ulnar side US of the hand, or *vice versa.* In an exemplary embodiment, the varying width B of the palm engaging portion 5 attains a maximum approximately near the middle of or halfway the palm engaging portion 5.

As can be seen from Figure 3, the (local) widening of the palm engaging portion 5 provides improved distributed support over a larger surface area of the palmar region 4 such that the palm engaging portion 5 reduces localized pressure points on the palm of the hand, thereby improving comfort. The (local) widening of the palm engaging portion 5 also allows for improved distributed support when a concave shape of the hand/palm is produced.

With regard to pinching the thumb T and index finger I, Figure 4 shows a lateral-radial view of a thumb brace 1 according to an embodiment of the present invention, wherein a pinching position/posture of the thumb T and index finger I is depicted. According to the present invention, the (local) widening of the palm engaging portion 5 through a varying width B thereof allows a patient to produce a natural round/oval shape "O" between the thumb T and index finger I whilst still providing orthopaedic support to the thumb T as required. When pinching the thumb T and index finger I as shown, the palmar region 4 is subjected to a distributed load through the widening palmar engaging portion 5, thereby increasing comfort.

In an embodiment, see Figure 3, the palm engaging portion 5 of the palmar wire section 3 comprises two wire parts 5a, 5b that are connected parallel/side-by-side at an ulnar side end e1 and opposing radial side end e2 of the palmar wire section 3, wherein the two wire parts 5a, 5b are separated, e.g. separate smoothly/continuously, in proximal-distal direction (PD-DD) between the ulnar and radial side ends e1, e2. This embodiment allows relatively narrow/thin wire parts 5a, 5b to provide sufficient distributed support to the palmar region 4 of a hand as the width B of the palm engaging portion 5 increases between the ulnar and radial side ends e1, e2, i.e. where the wire parts 5a, 5b separate at the ulnar side end e1 and subsequently join at the radial side end e2 as seen from the ulnar side US toward the radial side RS.

Note that the parallel/side-by-side connection of the two wire parts 5a, 5b at the radial side end e2 provides for a narrow web point W of the wire frame 2 that comfortably fits into the web space between the thumb T and the index finger I.

In an advantageous embodiment, the palm engaging portion 5 is a concave palm engaging portion, so that improved conformal engagement with a concave shaped palmar region 4 is facilitated. A concave palm engaging portion 5 further increases comfort and improves a snug fit of the thumb brace 1 to the hand, which in turn improves stability of the thumb wire section 11 providing support to the thumb T.

With reference to Figure 2 and Figure 5, in an embodiment the ulnar wire section 7 and the palmar wire section 3 are U-shaped wire sections, wherein the U-shape provides distributed support to the dorsal side of the hand and improves a snug and stable engagement of the thumb brace 1 with the hand.

In further embodiments, the ulnar wire section 7 is connected to the parallel connected wire parts 5a, 5b at the ulnar side end e1 and wherein the radial wire section 8 is connected to the parallel connected wire parts 5a, 5b at the radial side end e2. This embodiment allows a continuous wire frame 2 to be used wherein the two wire parts 5a, 5b seamlessly extend/bend into the ulnar wire section 7 and the radial wire section 8. Such a seamless design increases hygiene and stiffness of the wire frame 2. In case U-shaped ulnar and radial wire sections 7, 8 are used, then the wire frame 2 may be formed as a continuous loop of wire that smoothly bends to form the palmar wire section 3, e.g. the two wire parts 5a, 5b, the ulnar wire section 7 and the radial wire sections 8.

In an advantageous embodiment, the palmar wire section 3, the ulnar wire section 7 and the radial wire section 8 may form a single piece wire section. That is, a single piece/unitary wire section may be bent to form the palmar wire section 3 (e.g. the two wire parts 5a, 5b), the ulnar wire section 7 and the radial wire section 8 in continuous fashion so that a substantially rigid wire frame 2 is provided that has no sharp transitions that could comprise comfort and/or hygiene when wearing the thumb brace 1.

To further improve support to a thumb T for cases of e.g. MP hyperextension, there is an embodiment wherein the wire frame 2 further comprises a U-shaped thenar wire section 12 as shown in Figure 3. The thenar wire section 12 is connected to the palmar wire section 3 near/at the web point W thereof and configured to extend along a thenar eminence region 13 of the hand. The U-shaped thenar wire section 12 provides distributed support and stability to the thumb T, e.g. providing relief to the CMC joint of the thumb T. In an advantageous embodiment, the thenar wire section 12 forms a continuous wire section which is bent into a U-shape as shown, so that smooth engagement with the patient's thenar eminence region 13 is provided for added comfort.

As further depicted in Figure 3, in an embodiment the thenar wire section 12 comprises a first end 12a and a second end 12b, the first end 12a being connected to the palmar wire section 3 at the web point W and the second end 12b being connected to the thumb wire section 11. In this way the thenar wire section 12 provides rigidity to the thumb brace 1 between the palmar wire section 3 and the thumb wire section 11.

In a further embodiment, as exemplified in Figure 3, the first end 12a and the second end 12b of the thenar wire section 12 may be spatially separated, thereby providing an open end 12c to the U-shaped thenar wire section 12, wherein the open end 12c is located between the first end 12a and the second end 12b. The open end 12c allows for air circulation and room for skin to expand/shrink when e.g. pinching the thumb T and index finger I.

In an exemplary embodiment, the thumb wire section 11 and the thenar wire section 12 comprise or form a single piece/unitary wire section. In this embodiment a single piece or unitary piece of wire is bent forming the U-shaped thenar wire section 12 and the thumb wire section 11, thereby further eliminating sharp transitions/connections and as such provide improved smooth engagement with the skin.

To improve distributed support to the thump T, e.g. to the proximal phalanx portion thereof, an embodiment is provided wherein the thumb wire section 11 is shaped as a spiral/helix comprising at least one spiral turn, e.g. one and a half spiral turn. Such a spiral shape provides wider, distributed engagement of the thumb wire section 11 along the thumb T for reducing pressure points. The spiral shape allows a single/unitary piece of wire to be bent for forming the spiral shaped thumb wire section 11 and U-shaped thenar wire section 12 as a unitary wire section.

It is emphasized that the thumb brace 1 of the present invention comprises the wire frame 2 which is preferably made entirely of relatively narrow wire-like material, so the palmar wire section 3, the palm engaging portion 5 thereof (e.g. the two wire parts 5a, 5b), the ulnar wire section 7, the radial wire section 8, the thumb wire section 11 and the thenar wire section 12 all comprise such narrow wire-like material. To prevent uncomfortable pressure points to the skin, an advantageous embodiment is envisaged wherein the wire frame 2 is made of oval shaped wire, i.e. substantially flat wire having an oval(-like) cross section, thereby providing a larger surface area to all wire sections 3, 5, 5a, 5b, 7, 8, 11, 12 that engage the skin for improved comfort.

The wire frame 2 of the thumb brace 1 is preferably a metallic or metal alloy wire frame 2, to provide substantial rigidity to the wire frame 2 and in particular allow all wire sections 3, 5, 5a, 5b, 7, 8, 11, 12 to be obtained by shaping/bending the metal or metal alloy wire frame 2 into a desired shape.

As the thumb brace 1 is often worn over long periods of time, the wire frame 2, and in particular all wire sections 3, 5, 5a, 5b, 7, 8, 11, 12 thereof, preferably comprise silver, gold, bronze, titanium or a combination thereof, allowing the thumb brace 1 to be used in wet/moist environments and to minimize bacterial growth and dirt built-up.

It is interesting to note that in an alternative embodiment the thumb brace 1 of the present invention could be moulded as one unitary piece. That is, the entire wire frame 2 may be obtained through a moulding process instead of utilizing a particular length of wire from a roll, for example, and to deform and bend the wire to form the thumb brace 1. So in an embodiment, the wire frame 2 may be a single, unitary component, i.e. the palmar wire section 3, the ulnar wire section 7, the radial wire section 8 and the thumb wire section 11 may form a single, unitary component forming a unitary wire frame 2. The palmar wire section 3 then comprises a unitarily integrated palm engaging portion 5 that widens in proximal-distal direction.

In a further embodiment, the unitary wire frame 2 may further comprise the aforementioned locally separating wire parts 5a, 5b as a moulded unitary piece of the wire frame 2. The same applies for an embodiment wherein the unitary wire frame 2 further comprises the U-shaped thenar wire section 12 as a unitary moulded piece, wherein the thenar wire section 12 is unitarily connected/moulded with the palmar wire section 3 near/at the web point W thereof.

Therefore, the thumb brace 1, and in particular the entire wire frame 2, may be moulded as a unitary component so that the production process is simplified and manufacturing costs are lowered, wherein a metallic (e.g. silver) or plastic moulding material may be used.

In view of the above, the thumb brace 1 of the present invention can now be summarized by the following embodiments:
Embodiment 1. A thumb brace (1) for support of a thumb (T), comprising a wire frame (2) having
   a palmar wire section (3) configured to extend in lateral direction from a web point (W) thereof between a thumb (T) and index finger (I) along a palmar region (4) of the hand toward an ulnar side of the hand, wherein the palmar wire section (3) is connected to
   an ulnar wire section (7) and a radial wire section (8), the ulnar wire section (7) being configured to extend from the ulnar side of the hand in lateral direction along a dorsal ulnar region (9) of the hand and wherein the radial wire section (8) is configured to extend from the web point (W) along a first dorsal interosseous region (10), wherein the wire frame (2) further comprises
   a ring shaped thumb wire section (11) configured to receive the thumb (T), and wherein the palmar wire section (3) comprises a palm engaging portion (5) that widens in proximal-distal direction.
Embodiment 2. The thumb brace (1) according to embodiment 1, wherein the palm engaging portion (5) comprises two wire parts (5a, 5b) connected parallel at an ulnar side end (e1) and opposing radial side end (e2) of the palmar wire section (3), and wherein the two wire parts (5a, 5b) are separated in proximal-distal direction between the ulnar and radial side ends (e1, e2).
Embodiment 3. The thumb brace (1) according to embodiment 1 or 2, wherein the palm engaging portion (5) is a concave palm engaging portion.
Embodiment 4. The thumb brace (1) according to any one of embodiments 1-3, wherein the ulnar wire section (7) and the radial wire section (8) are U-shaped wire sections.
Embodiment 5. The thumb brace (1) according to any one of embodiments 1-4, when depending from claim 2, wherein the ulnar wire section (7) is connected to the parallel connected wire parts (5a, 5b) at the ulnar side end (e1) and wherein the radial wire section (8) is connected to the parallel connected wire parts (5a, 5b) at the radial side end (e2).
Embodiment 6. The thumb brace (1) according to any one of embodiments 1-5, wherein the palmar wire section (3), the ulnar wire section (7) and the radial wire section (8) form a single piece wire section.
Embodiment 7. The thumb brace (1) according to any one of embodiments 1-6, wherein the wire frame (2) further comprises a U-shaped thenar wire section (12) connected to the palmar wire section (3) at the web point (W) and configured to extend along a thenar eminence region (13) of the hand.
Embodiment 8. The thumb brace (1) according to embodiment 7, wherein the thenar wire section (12) comprises a first end (12a) and a second end (12b), the first end (12a) being connected to the palmar wire section (3) at the web point (W) and the second end (12b) being connected to the thumb wire section (11).
Embodiment 9. The thumb brace (1) according to embodiment 8, wherein the first end (12a) and the second end (12b) of the thenar wire section (12) are spatially separated.
Embodiment 10. The thumb brace (1) according to embodiment 8 or 9, wherein the thumb wire section (11) and the thenar wire section (12) form a single piece wire section.
Embodiment 11. The thumb brace (1) according to any one of embodiments 1-10, wherein the thumb wire section (11) is shaped as a spiral comprising at least one spiral turn.
Embodiment 12. The thumb brace (1) according to any one of embodiments 1-11, wherein the wire frame (2) is made of oval shaped wire.
Embodiment 13. The thumb brace (1) according to any one of embodiments 1-12, wherein the wire frame (2) is a metallic or metal alloy wire frame (2).
Embodiment 14. The thumb brace (1) according to embodiment 13, wherein the wire frame (2) comprises silver, gold, bronze, titanium or a combination thereof.
Embodiment 15. The thumb brace (1) according to any one of embodiments 1-14, wherein the wire frame (2) is a single, unitary component.

The present invention has been described above with reference to a number of exemplary embodiments as shown in the drawings. Modifications and alternative implementations of some parts or elements are possible, and are included in the scope of protection as defined in the appended claims.

## Claims

1. A thumb brace (1) for support of a thumb (T), comprising a wire frame (2) having
a palmar wire section (3) configured to extend in lateral direction from a web point (W) thereof between a thumb (T) and index finger (I) along a palmar region (4) of the hand toward an ulnar side of the hand, wherein the palmar wire section (3) is connected to
an ulnar wire section (7) and a radial wire section (8), the ulnar wire section (7) being configured to extend from the ulnar side of the hand in lateral direction along a dorsal ulnar region (9) of the hand and wherein the radial wire section (8) is configured to extend from the web point (W) along a first dorsal interosseous region (10), wherein the wire frame (2) further comprises
a ring shaped thumb wire section (11) configured to receive the thumb (T), and wherein the palmar wire section (3) comprises a palm engaging portion (5) that widens in proximal-distal direction.

2. The thumb brace (1) according to claim 1, wherein the palm engaging portion (5) comprises two wire parts (5a, 5b) connected parallel at an ulnar side end (e1) and opposing radial side end (e2) of the palmar wire section (3), and wherein the two wire parts (5a, 5b) are separated in proximal-distal direction between the ulnar and radial side ends (e1, e2).

3. The thumb brace (1) according to claim 1 or 2, wherein the palm engaging portion (5) is a concave palm engaging portion.

4. The thumb brace (1) according to any one of claims 1-3, wherein the ulnar wire section (7) and the radial wire section (8) are U-shaped wire sections.

5. The thumb brace (1) according to any one of claims 1-4, when depending from claim 2, wherein the ulnar wire section (7) is connected to the parallel connected wire parts (5a, 5b) at the ulnar side end (e1) and wherein the radial wire section (8) is connected to the parallel connected wire parts (5a, 5b) at the radial side end (e2).

6. The thumb brace (1) according to any one of claims 1-5, wherein the palmar wire section (3), the ulnar wire section (7) and the radial wire section (8) form a single piece wire section.

7. The thumb brace (1) according to any one of claims 1-6, wherein the wire frame (2) further comprises a U-shaped thenar wire section (12) connected to the palmar wire section (3) at the web point (W) and configured to extend along a thenar eminence region (13) of the hand.

8. The thumb brace (1) according to claim 7, wherein the thenar wire section (12) comprises a first end (12a) and a second end (12b), the first end (12a) being connected to the palmar wire section (3) at the web point (W) and the second end (12b) being connected to the thumb wire section (11).

9. The thumb brace (1) according to claim 8, wherein the first end (12a) and the second end (12b) of the thenar wire section (12) are spatially separated.

10. The thumb brace (1) according to claim 8 or 9, wherein the thumb wire section (11) and the thenar wire section (12) form a single piece wire section.

11. The thumb brace (1) according to any one of claims 1-10, wherein the thumb wire section (11) is shaped as a spiral comprising at least one spiral turn.

12. The thumb brace (1) according to any one of claims 1-11, wherein the wire frame (2) is made of oval shaped wire.

13. The thumb brace (1) according to any one of claims 1-12, wherein the wire frame (2) is a metallic or metal alloy wire frame (2).

14. The thumb brace (1) according to claim 13, wherein the wire frame (2) comprises silver, gold, bronze, titanium or a combination thereof.

15. The thumb brace (1) according to any one of claims 1-14, wherein the wire frame (2) is a single, unitary component.
